# EUROPEAN PATENT APPLICATION

(11) **EP 4 582 803 A2**
(43) Date of publication of application: **09.07.2025**
(21) Application number: 24220168.9
(22) Date of filing: 16.12.2024
(51) Int. Cl.: G01N 33/50, C07K 16/28, C12N 5/0783

(54) **POTENCY ASSAY BINDING COMPOSITIONS AND METHODS OF USE**

(30) Priority: 15.12.2023 US 202363610496 P
(71) Applicant: Stemcell Technologies Canada Inc., Vancouver, British Columbia V6A 1B6 (CA)
(72) Inventor: EWEN, Catherine, Vancouver V6A 1B6 (CA); YU, Jie, Vancouver V6A 1B6 (CA)
(74) Representative: Mathys & Squire

(57) **Abstract**

The present disclosure relates to potency assays for eliciting effector function of effector cells, and more specifically to potency assays for eliciting effector function of effector cells by physical linkage to target cells. Disclosed methods include co-culturing an effector cell displaying a first antigen and a target cell displaying a second antigen in the presence of a binding composition that physically links the effector cell and the target cell to bring the cells into close proximity and elicit the effector function of the effector cell. Methods and compositions of this disclosure may be used to enhance an effector function of effector cells upon target cells in assays such as cell potency or cell killing assays.

## Description

### CROSS-REFERENCE TO RELATED APPLICATION

This application claims the benefit of United States Provisional Patent Application No. 63/610,496, filed December 15, 2023, the entire contents of which is hereby incorporated by reference in its entirety.

### TECHNICAL FIELD

This disclosure relates to cell-based assays, and more specifically to potency assays for eliciting effector function of effector cells, and still more specifically to potency assays for eliciting effector function of effector cells through specific linking to target cells.

### BACKGROUND

There is a need to assess functional of immune cells in various immunological applications ranging from research tools to cell-based therapies, particularly immune cell therapies. Measuring potency of cell therapies is challenging due to variability, and the need for well-defined, reliable, and reproducible assays. Potency assays are becoming a key requirement of cell therapy regulators as part of release criteria and quality control measures.

While potency assays typically rely on the stochastic interaction of target cells and effector cells lending to assay variability, there is a need for methods to assess the potency of immune cells while addressing the problems of current methods, such as cost, lack of a flexible platform that with minor modifications can be used for any combination of effector and target cells, and scalability. Common potency assays are also limited in their applicability to diverse effector-target cell types. For instance, antibody dependent cellular cytotoxicity (ADCC) and antibody dependent cellular phagocytosis (ADCP) assays require effector cells expressing a crystallizable fragment (Fc) receptor that interacts with the Fc portion of antibodies. Potency not so constrained could be useful in expanding the repertoire of effector cells and/or target cells that could be tested.

Therefore, a reliable and reproducible way to assess potency may satisfy the requirements of regulators. Thus, novel binding compositions and their uses in methods as disclosed herein may offer better control over immune cell-based product potency and provide increased biological relevance.

### SUMMARY

In one aspect of this disclosure are provided methods of eliciting an effector function of an effector cell. Methods of this disclosure may comprise co-culturing an effector cell displaying a first antigen and a target cell displaying a second antigen in the presence of a binding composition, and linking the effector cell and the target cell via the binding composition to one another to elicit the effector function of the effector cell. Binding compositions are not particularly limited that they can bind each of a target cell and an effector cell and bring them into close proximity.

A binding composition may comprise at least one first recognition motif of a first (one or more) polypeptide member that binds a first antigen of an effector cell and at least one second recognition motif of a second (one or more) polypeptide member that binds a second antigen of a target cell. A first antigen may be or may be comprised in CD3, CD16, CD56, CD335/NKp46 or a receptor on the surface of the effector cell. A second antigen may be or may be comprised in CD19, a tumor antigen or a receptor on the surface of the target cell.

A first polypeptide member and a second polypeptide member may either be directly linked or indirectly linked. In one embodiment, a first polypeptide member and a second polypeptide member are directly linked. In one embodiment, a first polypeptide member and a second polypeptide member are indirectly linked. If indirectly linked, a first polypeptide member and a second polypeptide member may be indirectly linked via a scaffold, a bead, a particle or at least one third polypeptide member.

A first polypeptide member and/or a second polypeptide member may be an antibody, antibody fragment, an immunoglobulin or a protein.

An effector cell is typically an immune cell, preferably a T cell or any T-cell subtype, an NK cell or any NK cell subtype, or an NKT cell. In one embodiment, an effector cell does not comprise an Fc receptor. In one embodiment, an effector cell comprises an Fc receptor.

A target cell may be a cell or a mass of cells. In one embodiment, a mass of cells is a tumor, an organoid or a spheroid.

An effector function of an effector cell may comprise killing a target cell or activation of an effector or target cell. An effector cell may kill a target cell by degranulation or by death receptor-ligand interactions.

Target cells and effector cells may be co-cultured for a time period sufficient to a) link the effector cell and the target cell, and/or b) elicit the effector function of the effector cell. In one embodiment, the time period ranges between 0.5 to 24 hours. In one embodiment, an effector cell and a target cell are linked via a binding composition prior to co-culturing. In one embodiment, co-culturing an effector cell and a target cell further comprises IL-2, IL-15, IL-21 or a combination thereof.

Methods of this disclosure may further comprise measuring an effector function of an effector cell by flow cytometry, marker expression analysis, cytokine production, protein analysis, transcriptomic analysis or metabolomic analysis of target cells.

Linking an effector cell and a target cell via a binding composition may enhance an effector function of the effector cell by at least 5%, at least 10%, at least 20%, at least 30% or more compared to when an effector cell and a target cell are co-cultured but not linked via the binding composition.

In one embodiment, the effector cells are present in a ratio of at least 1:1 with the target cells.

In one embodiment, the target cell and/or the effector cell are adherent cells or suspension cells.

In another aspect of this disclosure are provided compositions comprising a first recognition motif of a first polypeptide member that binds a first antigen of an effector cell; and a second recognition motif of a second polypeptide member that binds a second antigen of a target cell, wherein the first polypeptide member and the second polypeptide member are indirectly linked. A first antigen may be or may be comprised in CD3, CD16, CD56, CD335/NKp46 or a receptor on the surface of the effector cell. A second antigen may be or may be comprised in CD19, a tumor antigen or a receptor on the surface of the target cell.

Compositions of this disclosure may further comprise: (i) a scaffold, a bead or a particle; and/or (ii) at least a third polypeptide member, to indirectly link a first polypeptide member and a second polypeptide member.

A first polypeptide member and/or a second polypeptide member may an antibody, an antibody fragment, an immunoglobulin or a protein.

A first polypeptide member and a second polypeptide member may each be present in a binding composition at a concentration within the range of 10 -1000 ng/ml. In one embodiment, the first polypeptide member, the second polypeptide member and the third polypeptide member are each present at a concentration within the range of 10 -1000 ng/ml. In one embodiment, the first polypeptide member and the second polypeptide member are each present at a concentration within the range of 30 -600 ng/ml. In one embodiment, the first polypeptide member, the second polypeptide member and the third polypeptide member are each present at a concentration within the range of 30 -600 ng/ml.

### BRIEF DESCRIPTION OF THE DRAWINGS

For a better understanding of the various embodiments described herein, and to show more clearly how these various embodiments may be carried into effect, reference will be made, by way of example, to the accompanying drawings which show at least one example embodiment, and which are now described. The drawings are not intended to limit the scope of the teachings described herein.

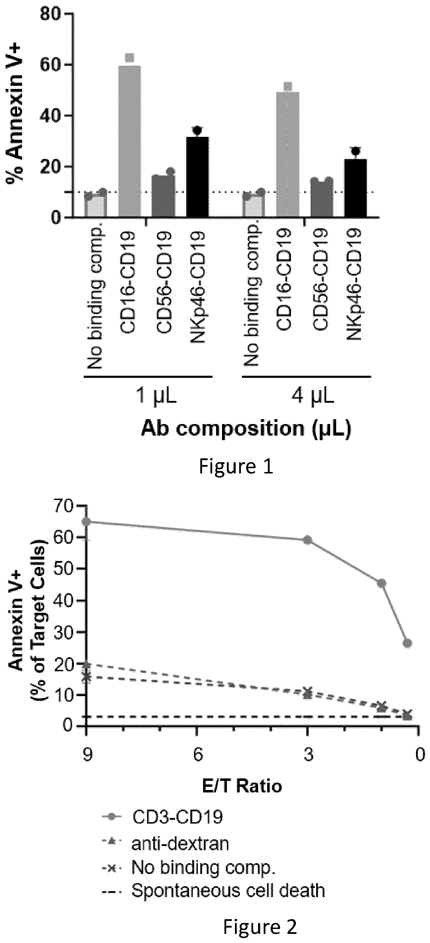

Figure 1 shows a bar graph quantifying apoptotic cells following a killing assay carried out by linking effector cells to target cells using binding compositions of this disclosure. Effector NK cells were co-cultured with target NALM 6 cells at a 3:1 effector to target (E/T) ratio, and were linked using various binding compositions at different concentrations (1 µL and 4 µL). The percent positive Annexin V target cells were measured by flow cytometry. Binding compositions comprising a first polypeptide member (e.g., an antibody) that binds an NK cell antigen (e.g. CD16, CD56 or NKp46) indirectly linked to a second polypeptide member (e.g., antibody) that binds a target antigen (e.g., CD19), denoted by CD16-CD19, CD56-CD19 and NKp46 were used. Controls included co-cultured cells without any binding composition (No binding comp.). Data represent the mean of 1-2 experiments. Baseline levels of % Annexin V⁺ cells without any binding composition is shown with a black dotted line.
Figure 2 shows quantification of apoptotic cells following a killing assay carried out by linking effector cells to target cells using binding compositions of this disclosure. Effector Pan T cells expanded for 10 days were co-cultured with target NALM 6 cells at various E/T ratios, as indicated. Killing was enhanced by linking effector and target cells using a binding composition comprising a first polypeptide member (e.g., an antibody) that binds a T cell antigen (e.g. CD3) indirectly linked to a second polypeptide member (e.g., antibody) that binds a target antigen (e.g., CD19), denoted by CD3-CD19 (each polypeptide member at a concentration of 0.025 µg/ml). Target cell death was measured based on % Annexin V⁺ cells assessed by flow cytometry and plotted against varying E/T ratios of 9:1, 6:1 and 3:1. Controls included effector and target cells co-cultured either without a binding composition (denoted as No Ab) or in the presence of a binding composition comprising a monospecific complex of irrelevant (anti-dextran) antibodies (denoted as dextran-dextran). Spontaneous cell death is shown with a black dotted line. Data represents the mean of 2 technical replicates from 1 experiment.

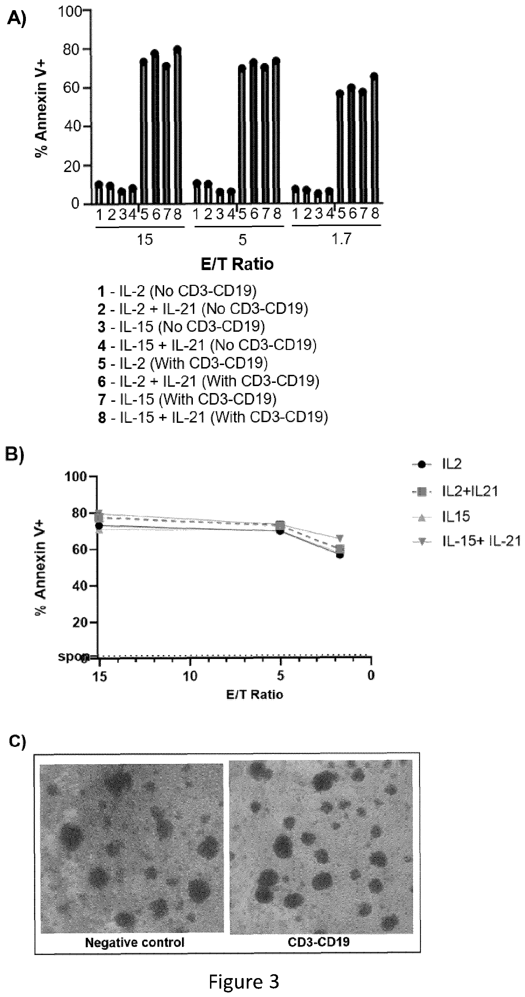

Figure 3 shows quantification of apoptotic cells following a killing assay carried out by linking effector cells to target cells using binding compositions of this disclosure in the presence of different cytokines (IL-2, IL-15 and/or IL-21). Effector T cells expanded for 12 days were co-cultured with target NALM 6 cells at various E/T ratios of 15, 5 and 1.7, as indicated. A binding composition comprising a first polypeptide member (e.g., an antibody) that binds a T cell antigen (e.g. CD3) indirectly linked to a second polypeptide member (e.g., antibody) that binds a target antigen (e.g., CD19), denoted by CD3-CD19, was used. Killing of target cells was enhanced by linking effector and target cells in the presence of the binding composition in comparison to an absence of the binding composition (A). Target cell death was measured based on % Annexin V⁺ cells assessed by flow cytometry. The percentage of Annexin V⁺ target NALM-6 cells after co-culture of Day 12 expanded effector T cells with target NALM-6 cells in the presence of specific cytokines (IL-2, IL-2+IL-21, IL-15 and IL-15+IL-21) and of the binding composition (CD3 -CD19) was tested with varying E/T ratios of 15, 10, 5 and 0 (B). Spontaneous cell death is shown with a black dotted line (B). Bright field microscopy-based analysis of co-culture of effector T cell and targetNALM-6 cells in the presence of IL-2 and at an E/T ratio of 15:1, with and without a binding composition ( CD3 -CD19 ) was performed (representative image shown)(C).

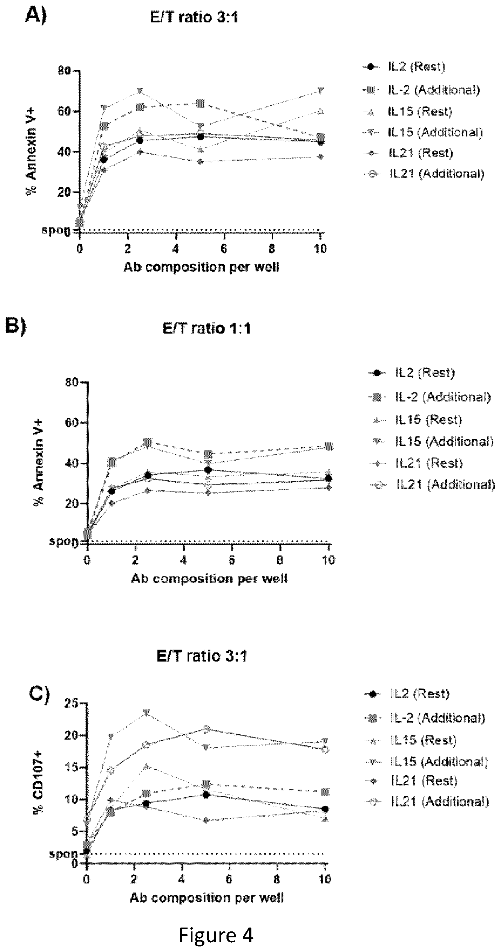

Figure 4 shows quantification of apoptotic cells following a killing assay carried out by linking effector cells to target cells using binding compositions of this disclosure in the presence of different cytokines. Effector CD8⁺ T cells expanded for 8 days were co-cultured with target NALM 6 cells at various E/T ratios, as indicated, and with cytokine addition at different times, also as indicated. Killing was enhanced by linking effector and target cells in the presence of a binding composition comprising a first polypeptide member (e.g., an antibody) that binds a T cell antigen (e.g. CD3) indirectly linked to a second polypeptide member (e.g., antibody) that binds a target antigen (e.g., CD19). Cytokines were added either 2-days prior to co-culture (Additional) or over the duration of co-culture (Rest). Target cell death was measured based on % Annexin V⁺ cells assessed by flow cytometry and plotted against varying volumes (µL/ml) of antibody composition per well. Spontaneous cell death is shown with a black dotted line (A & B). Cytotoxic degranulation of the CD8⁺ T cells was assessed by flow cytometry based on membrane expression of the lysosomal protein CD107 plotted against varying volumes (µL/ml) of antibody composition per well (C).

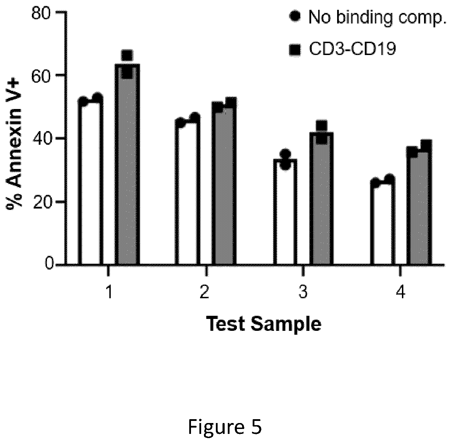

Figure 5 shows a bar graph quantifying apoptotic cells following a killing assay carried out by linking effector cells to target cells using binding compositions of this disclosure. Effector CD8⁺ T cells differentiated from pluripotent stem cells (PSCs) were co-cultured with target NALM 6 cells. Killing was enhanced by linking effector and target cells in the presence of a binding composition comprising a first polypeptide member (e.g., an antibody) that binds a T cell antigen (e.g., CD3) indirectly linked to a second polypeptide member (e.g., antibody) that binds a target antigen (e.g., CD19), denoted as CD3-CD19. Target cell death was measured based on % Annexin V⁺ cells assessed by flow cytometry and plotted against 4 test samples corresponding to 4 different differentiations of H9 PSC cell line to obtain CD8⁺ T cells. Data represents the mean of 2 technical replicates from 1 experiment for each condition.

### DETAILED DESCRIPTION

This disclosure relates to methods and composition for assessing potency and/or functionality of cells, such as by eliciting an effector function of an effector cell upon a target cell. By physically linking the effector and target cells using a binding composition, potency assays may be improved by forcing close association of relevant cell types rather than relying on stochastic interaction, such as by passive diffusion.

Where used in this disclosure, the term "immune cell" refers to one of various types of cells of the immune system. Immune cells may comprise any type of immune cell such as T lymphocytes, Natural killer (NK) cells, B lymphocytes, monocytes, granulocytes, T helper cells, basophils, dendritic cells, neutrophils, cytotoxic T cells, Langerhans cells or microglia. In one embodiment, immune cells are T lymphocytes or any subset thereof, such as regulatory T cells, helper T cells, cytotoxic T cells, gamma delta (γδ) T cells, natural killer T (NKT) cells, memory T cells or Chimeric Antigen Receptor (CAR) T cells. In one embodiment, immune cells are Natural killer (NK) cells or any subset thereof, such as NK progenitor cells, immature NK cells, mature NK cells, tolerant NK cells, cytotoxic NK cells or regulatory NK cells. Immune cells of this disclosure may be obtained/isolated/enriched directly from patients or donors, may be purchased from a commercial supplier, or may be differentiated from a stem cell (e.g. a pluripotent stem cell or a hematopoietic stem cell, or a progenitor thereof). Immune cells may be derived from a mixed population of cells such as lymph, whole blood or peripheral blood mononuclear cells (PBMCs).

Where used in this disclosure, the term "binding composition" refers to a binding composition comprising a first recognition motif of a first polypeptide member that binds a first antigen and a second recognition motif of a second polypeptide member that binds a second antigen. A binding composition may comprise any antigen-binding composition known in the art and may comprise at least a first recognition motif of a first polypeptide member that binds a first antigen and a second recognition motif of a second polypeptide member that binds a second antigen. A first polypeptide member and a second polypeptide member may preferably be present as separate components of a binding composition, which may or may not be linked, whether directly or indirectly. Examples of a polypeptide member include, without limitation, antibodies (e.g., monoclonal or polyclonal), antibody fragments (e.g., Fab or F(ab)₂ fragments), immunoglobulins, bi-specific antibodies, antibody complexes, antibody mimetics, antibody variants, labeled antibodies, single-domain antibodies, single-chain antibodies, multi-specific antibodies, antibody fusion proteins, immunoadhesins and antigen binding molecules/proteins/ peptides capable of recognizing one or more antigen displayed on the surface of an effector and/or a target cell. Antibodies may comprise any isotype known in the art such as IgA, IgG, IgE, IgD or IgM. Binding compositions may either be derived from naturally occurring sources (natural binding compositions) or may be generated by synthetic or artificial means (synthetic binding compositions). Natural binding compositions may comprise antibodies produced by antibody-secreting cells such as B lymphocytes and may be derived from humans, birds or animals such as mice, rabbits, sheep, chickens, rats, hamsters, guinea pigs, goats, donkeys, cows, camels or llamas. Synthetic binding compositions may be generated in vitro, without the use of animals and may include, without limitations, recombinant antibodies, nucleic acid aptamers and non-immunoglobulin protein scaffolds. Binding compositions (e.g., natural or synthetic) may have binding specificities against one or more than one recognition motif of an effector or a target cell. Such binding specificities may be engineered to bind or recognize any desired target recognition motif and may extend beyond the natural immune repertoire. Antibody mimetics may refer to molecules that are not generated by an immune system and are not structurally related to antibodies but can recognize and bind antigens similar to antibodies.

Where used in this disclosure, the term "recognition motif" refers to the region of a binding composition that recognizes and binds/interacts with an antigen, as may be present on an effector and/or a target cell. In some embodiments, the term "recognition motif" may refer to a binding domain or a paratope of an antibody, an antibody fragment, an immunoglobulin, an aptamer, a protein, an antigen binding molecule or an antigen binding peptide.

Where used in this disclosure, the term "polypeptide member" refers to one of potentially a plurality of distinct components of a binding composition of this disclosure. A polypeptide member may be or be comprised in an antibody, an antibody fragment, an antibody mimetic, an immunoglobulin, a protein, a polypeptide, a peptide, an aptamer, or any other antigen binding molecule or compound.

Where used in this disclosure, the term "directly linked" refers to conjugation, association or attachment of a first polypeptide member (e.g., an antibody) and a second polypeptide member (e.g., an antibody) to one another. A first polypeptide member and a second polypeptide member may be directly linked by a chemical conjugation method known in the art. A first polypeptide member and a second polypeptide member may be directly linked by being comprised in a bispecific antibody, wherein at least one or more variable regions thereof are engineered to bind different/multiple antigens, such as a first antigen of an effector cell and a second antigen of a target cell.

Where used in this disclosure, the term "indirectly linked" refers to linking of the first polypeptide member (e.g., an antibody) and a second polypeptide member (e.g., an antibody) via indirect means, and the association of the polypeptide members may be intermediated by one or more members or components. A first polypeptide member and a second polypeptide member may be indirectly linked via a scaffold, a bead, a particle or at least a third polypeptide member.

Where used in this disclosure, the term "effector cell" refers to any of various types of cells that may actively respond to a stimulus and effect a change in either itself or through paracrine signaling. Types of changes may include, but are not limited to, induction of release of cytokines, chemokines or antibodies, release of granules or cytotoxic/effector molecules or proteins, activation of macrophages or modifying activity of accessory cells. An effector function of an effector cell may be imparted upon a target cell.

Where used in this disclosure, the term "target cell" refers to any of various types of cells upon which effector cell functions are imparted. Types of changes may include, but are not limited to, induction of apoptosis, cell death, induction of release of cytokines, chemokines or antibodies, activation of receptors, induction of signaling or transcription pathways and induction of mRNA or protein expression.

Where used in this disclosure, the term "pluripotent stem cell" or "PSC" refers to a cell capable of self-renewal and of differentiation into specialized cell types of the three germ layers (ectoderm, endoderm and mesoderm). PSCs may refer to both embryonic stem cells (ESCs) and induced pluripotent stem cells (iPSCs). Stem cells may be isolated, derived or induced from any source species, but in this disclosure, stem cells are preferably mammalian.

### Methods

In one aspect of this disclosure are provided methods of eliciting an effector function of an effector cell. Methods of this disclosure may more reliably elicit effector functions of an effector cell upon a target cell by forcing their physical linkage using a binding composition of this disclosure.

Effector functions of effector cells are too numerous to list exhaustively here, although in general effector functions of effector cells may comprise killing a target cell, activating an effector cell, or activating a target cell. Regardless, the breadth of effector functions would be apparent to one ordinarily skilled in the art.

If an effector function involves killing a target cell, killing may be effected by activating apoptotic pathways such as through death receptors (e.g. Fas, TNFαR, DR3, DR4, and/or DR5 by their respective death receptor ligands). Killing via death receptor pathways may comprise recruitment of specialized adaptor proteins and activation of caspase-based signaling cascades, or killing may occur in the absence of caspase activation. In addition or in the alternative, killing function of an effector cell may be carried out via degranulation (e.g. a release of cytotoxic proteins, such as granzyme B, from effector cell granules).

If an effector function involves activating effector cells, activation may comprise inducing production of cytokines, such as Interferon-gamma and/or TNF-alpha, expression of surface markers such as 4-1BB (CD137) or CD69, changes in effector cell transcriptomes or metabolomic changes in effector cells.

If an effector function involves activating a target cell, activation may comprise inducing release of cytokines, chemokines or antibodies, activation of receptors, induction of signaling or transcription pathways and induction of mRNA or protein expression.

Effector function of an effector cell may also be carried out by activating a target cell (e.g., immune cells) and cytotoxic degranulation. Such immune cell activation and cytotoxic degranulation may be assessed by membrane or surface expression of lysosomal-associated proteins that are typically present on the lipid bilayer surrounding lytic granules. An example of such a lysosomal-associated protein may include CD107a.

Methods of this disclosure may comprise co-culturing an effector cell displaying a first antigen and a target cell displaying a second antigen in the presence of a binding composition of this disclosure.

An effector cell and a target cell may be from the same species or from different species. an effector cell and/or a target cell may be human or non-human. In some embodiments, an effector cell may be human and a target cell may be non-human, or vice versa.

An effector cell and/or a target cell may be an adherent cell or a suspension cell. In some embodiments, an effector cell may be an adherent cell and a target cell may be a suspension cell or vice versa.

An effector cell of this disclosure is not particularly limited, provided that such a cell brings about some change in or effect upon a target cell, whether through direct interaction or through paracrine signaling. Typically, an effector cell may be one of a variety of immune cells, such as a T cell or a T cell subtype, NK cells or a NK cell subtype, or B cells or a B cell subtype. Specific examples of effector T cells include but are not limited to T cell, gamma/delta T cells, Regulatory T cells (e.g. Tr1 cells), chimeric antigen receptor (CAR) T cells, T helper cells (e.g. Th1 cells, Th2 cells, Th17 cells), cytotoxic T cells, memory T cells, CD4 T cells, CD8 T cells and CD127 cells Specific examples of effector NK cells include but are not limited to NK cells, CAR NK cells, NK progenitor cells, immature NK cells, mature NK cells, tolerant NK cells, cytotoxic NK cells or regulatory NK cell. Specific examples of effector B cells include but are not limited to transitional B cells, follicular B cells (FoB or B-2), marginal zone B cells (MZB), B1 B cells, mature peripheral B cells, plasmocyte or antibody-secreting cells, memory B cells or regulatory B cells (Bregs). Other potential types of effector cells may include monocytes, granulocytes, basophils, dendritic cells, neutrophils, microglia, macrophages or tumor infiltrating lymphocytes (TILs).

An effector cell may not comprise an Fc receptor. An Fc receptor may recognize Fc fragment of antibodies. Therefore, methods of this disclosure may be used to bring an effector and a target cell into close physical contact independent of the expression of a Fc receptor by one or both of an effector cell or a target cell. Methods of this disclosure may therefore provide a significant advantage over ADCC and ADCP assays known in the art which are reliant on an interaction between Fc domain of an antibody and an Fc receptor present on an effector or a target cell. Methods of this disclosure may not require any modification to an Fc domain comprised in a binding composition (e.g. an antibody) to modulate interactions with an Fc receptor of an effector or a target cell.

A target cell of this disclosure is not particularly limited, provided that such a cell exhibits sensitivity to an effector function of an effector cell. A target cell may be an individual cell or may be comprised in a mass of cells. If an individual cell, a target cell could be any cell that is acted upon by an effector cell, such as another immune cell, a tumour cell, a diseased cell, an infected cell, a model cell line (as may be used in a potency assay of a cell therapy product). Certain target cells may express at least one tumor-associated antigen (e.g. EGFR, Her2, CD19 or EpCAM). Examples of cell lines that may be used as a target cell include, but are not limited to, A549, SKBR3, SKOV3 or MCF-7. An exemplary target cell that is a model cell line is a leukemia cell line (e.g. NALM-6, K562). A NALM-6 cell line may be well known in the art as a B cell precursor leukemia cell line commonly used in immune-oncology research. A NALM-6 cell may express a tumor antigen CD194 or a tumor antigen CD19.

If comprised in a mass of cells, the mass of cells may be a tumour, an organoid, a tumour organoid or a spheroid.

An antigen of this disclosure, whether referring to a first antigen or a second antigen, is not particularly limited and may comprise any known or unknown antigen provided that such antigen may become bound by a binding composition of this disclosure. An antigen may be or comprise any macromolecule (e.g. protein, peptide, polysaccharide, carbohydrate, lipid or nucleic acid) moiety, allergen or foreign substance, provided it may be bound by a recognition motif (e.g. antibody) of a binding composition, as described herein. An antigen may be naturally present on a cell or may be exogenous to a cell. Where an antigen is exogenous to a cell, it may be derived from a source outside of the cell such as from viruses, parasites, fungi, bacteria, tumor cells or other cellular sources apart from the cell carrying the exogenous antigen.

A binding composition of this disclosure is not particularly limited, provided that it physically links an effector cell and a target cell by interacting with at least one antigen on the effector and at least one antigen of the target cell.

A binding composition of this disclosure may comprise a first polypeptide member and a second polypeptide member. A binding composition may comprise a first recognition motif of a first polypeptide member that binds a first antigen of an effector cell and a second recognition motif of a second polypeptide member that binds a second antigen of a target cell.

A first polypeptide member and/or a second polypeptide member may be an antibody, an antibody fragment, an antibody mimetic, an immunoglobulin, a peptide or a protein.

A first polypeptide member and a second polypeptide member may be directly linked. In some embodiments, a binding composition may comprise a first polypeptide member and a second polypeptide member that are directly linked. A first polypeptide member and a second polypeptide member may be directly linked by a chemical conjugation or a crosslinking method known in the art. Such a crosslinking method may involve crosslinking agents/molecules that comprise two or more reactive ends that are capable of chemically attaching to specific functional groups such as primary amines, sulfhydryl etc on a polypeptide member (e.g., proteins or other molecules). Conjugation methods may involve N-terminus, C-terminus, lysine residues, cysteine disulfide bonds present on a polypeptide member. Conjugation methods are not particularly limited and may comprise any method including, but not limited to, conjugation mediated by glutaraldehyde, reductive amination, N-Hydroxysuccinimide (NHS) ester-maleimide, thiolation agent or immunotoxins. A first polypeptide member and a second polypeptide member may be directly linked by recombinant molecular biology or nucleic acid-based approaches. A first polypeptide member and a second polypeptide member may be directly linked by being comprised in a bispecific antibody, wherein at least one or more variable regions thereof are engineered to bind different/multiple antigens, such as a first antigen of an effector cell and a second antigen of a target cell. A bispecific antibody may also be derived by linking two single-chain variable fragments (scFVs) with a specificity for two different antigens (e.g., a first antigen and a second antigen).

A first polypeptide member and a second polypeptide member may be indirectly linked. A first polypeptide member and a second polypeptide member may be indirectly linked via a scaffold (e.g. a macromolecule, such as a protein), a bead (e.g. a magnetic particle or nanoparticle), a particle or at least one third macromolecule, such as a polypeptide member.

Where a first polypeptide member and a second polypeptide member are indirectly linked via at least one third polypeptide member, linkage of the first polypeptide member and the second polypeptide may be intermediated by at least one or two third polypeptide member. If a first polypeptide member and a second polypeptide member of a binding composition are indirectly linked via at least one third polypeptide member reliant on antigen: recognition motif interaction, then an arising binding composition may comprise polypeptide members indirectly linked or held in an immunological complex or array. In a specific, and non-limiting embodiment, a first polypeptide member and a second polypeptide member are indirectly linked via at least one third polypeptide member, wherein the first and second polypeptide members comprise a full or partial Fc fragment and the at least one third polypeptide member comprises at least two recognition motifs that bind the full or partial Fc fragment of the first and second polypeptide members.

A binding composition comprising at least a first polypeptide member (comprising a first recognition motif) and a second polypeptide member (comprising a second recognition motif) may be bispecific, that is the first recognition motif and the second recognition motif have or possess different binding specificities. Or, a binding composition comprising at least a first polypeptide member (comprising a first recognition motif) and a second polypeptide member (comprising a second recognition motif) may be monospecific, that is the first recognition motif and the second recognition motif have or possess the same or similar binding specificities.

Thus, a binding composition of this disclosure may physically link an effector cell and a target cell via first and second polypeptide members (more particularly via first and second recognition motifs thereof), and such physical linkage may elicit an (enhanced) effector function of an effector cell upon a target cell. An enhanced effector function of an effector cell upon a target cell may be expressed relative a baseline level of effector function obtained when co-culturing a target cell and an effector cell in the absence of a binding composition of this disclosure.

Where an effector cell is a T cell or a subtype thereof, a first antigen of the T cell or subtype thereof may be one of various cell surface markers, proteins, glycoproteins, receptors, or epitope thereof expressed/displayed by such cells. Examples of a first antigen of a T cell or a subtype thereof may include but are not limited to CD3, CD4, CD8, CCR4/CD194, CCR5/CD195, CCR6/CD196, CCR7/CD197, CCR10, CD127, CD27, CD28, CD38, CD45 isoform (CD45RA or CD45RO), CD58/LFA3, CD69, CTLA4/CD152, CXCR3/CD183, FAS/CD95, HLA-DR, IL2RA/CD25, IL2RB/CD122, ITGAE/CD103, ITGAL/CD11a, KLRB1/CD161, NCAM1/CD56, PECAM1/CD31, PTGDR2/CD294/CRTH2 or SELL/CD62L/L-Selectin. In a specific embodiment, a first antigen is or is comprised in CD3, CD16, CD56, CD335/NKp46 or any receptor expressed on the surface of a T cell or a subtype thereof.

Where an effector cell is an NK cell or a subtype thereof, a first antigen of the NK cell or subtype thereof may be one of various cell surface markers, proteins, glycoproteins, receptors, or epitope thereof expressed/displayed by such cells. Examples of a first antigen of an NK cell or subtype thereof may include but are not limited to CD56, CD94, CD122/IL-2 R beta, CD244, NK1.1, NCR1, Ly49, CD49b, CD11b, KLRG1, CD27, CD16, KIR family receptors, NKG2A, NKG2D, NKp30, NKp44, NKp46, NKp80 or an Fc receptor. In a specific embodiment, a first antigen is or is comprised in CD16, CD56, CD335/NKp46 or any receptor expressed on the surface of a NK cell, including an Fc receptor.

A second antigen, as displayed by a target cell of this disclosure, may be one of various cell surface markers, proteins, glycoproteins, receptors, or epitope thereof expressed/displayed by such cells. Examples of a second antigen may may include but are not limited to CD3, CD4, CD8, CD19, CD20, CD33, CD52, CD146 or CD326. Examples of a second antigen of a target cell include but are not limited to glycoproteins, integrins, mucins, MHC receptors, ion channel proteins, lectins, TNF receptor superfamily proteins, cellular adhesion proteins, growth factor receptors or gangliosides. In a specific embodiment, a second antigen is or is comprised in CD19, a tumor antigen, a tumor-associated antigen (TAA), or any receptor expressed on the surface of a target cell.

Methods of this disclosure may comprise co-culturing effector cells and target cells under conditions amenable to effector cells eliciting effector function(s) upon target cells. Non-limiting variables of co-culture conditions include culture duration, culture media and additives thereto, sequence of events, cell densities, effector to target (E/T) cell ratios, and binding composition concentration.

With regard to culture duration, a co-culture of this disclosure will be for at least a time period sufficient to a) link an effector cell and a target cell, and b) to elicit an effector function of the effector cell. In some embodiments, the time period may be 10 minutes, 20 minutes, 30 minutes, 40 minutes, 50 minutes or 60 minutes. In some embodiments, the time period may be 1 hour, 2 hours, 3 hours, 4 hours, 5 hours, 6 hours, 7 hours, 8 hours, 9 hours, 10 hours, 11 hours or 12 hours. In one embodiment, the time period may range from 0.5 hours to 24 hours.

In some embodiments, an effector cell displaying a first antigen and a target cell displaying a second antigen may be linked via a binding composition comprising a first recognition motif of a first polypeptide member that binds the first antigen and a second recognition motif of a second polypeptide member that binds the second antigen prior to co-culturing the effector cell and the target cell. Such a pre-linking step as described above may be performed under *in vitro* conditions prior to initiating co-culture of the target and effector cells.

With regard to culture media, co-culture media are any media that support an effector cell eliciting an effector function upon target cells. If effector cells and target cells have different nutritional, metabolic, and/or cytokine or growth factor needs, then it may be important to determine/consider whether it is preferable that the assay is performed in media that better support the effector cells or the target cells. In an ideal scenario, a single media formulation at least adequately supports both effector cells and target cells. Exemplary co-culture media include, but are not limited to, ImmunoCult^{™} branded media (STEMCELL Technologies).

Another consideration regarding co-culture and co-culture media is the duration of time that effector cells and target cells are co-cultured in order to elicit effector function(s) of effector cells. If the duration of time is relatively short (e.g. less than 24 hours, or less than 12 hours, or 6 hours or less), then it may not matter how well a single medium supports both effector and target cells, provided that such medium does not itself result in an unacceptable loss of either cell type or a change in its function. In one embodiment, an ability of a co-culture medium to merely maintain one or both of effector cells and target cells in culture may be sufficient to elicit and analyze effector functions.

Still relating to co-culture conditions, methods of this disclosure may comprise culturing effector cells and target cells separately in a cell-specific culture medium prior to co-culturing the effector cells and the target cells (in a co-culture medium). Thus, cell-specific culture media of this disclosure may be any media that support the growth, maintenance, expansion, activation or differentiation of effector cells and target cells. In embodiments where the effector cells are immune cells such as T cells or NK cells, then such cells may be cultured in an appropriate ImmunoCult^{™}, StemSpan^{™}, or StemDiff^{™} medium (all STEMCELL Technologies). Likewise, depending on the nature of the target cells, they may be cultured in an appropriate cell-specific medium.

In one embodiment, a cell-specific media used to culture either effector cells or target cells may be used as a co-culture medium. In a specific embodiment, a co-culture medium may be an ImmunoCult^{™}-branded medium (STEMCELL Technologies).

A co-culture medium may be further supplemented with one or more of: cytokines, growth factors, small molecules, serum/albumin/serum replacement, proteins, lipids, etc. In one embodiment, a co-culture medium comprises one or more (additional) cytokines, such as IL-2, IL-7, IL-15, IL-21, or any combination thereof.

Whether a cell-specific or a co-culture medium, such media will comprise a basal medium typically comprising one or more of: amino acids, vitamin(s), organic and/or inorganic salt(s), buffer(s), antioxidant(s), protein(s), energy (e.g., carbon) source(s), and the like for supporting the growth of cells. In some embodiments, basal media do not include one or more of the foregoing components, and if essential may be supplemented. Numerous basal media are known and commercially available, including Dulbecco's Modified Eagle's Medium (DMEM), F12, Roswell Park Memorial Institute Medium (RPMI) 1640, Iscove's Modified Dulbecco's Medium (IMDM), Advanced DMEM, Advanced DMEM/F-12, Immunocult^{™}-branded basal media, and various others marketed specifically for the culture of immune cells. In one embodiment, the basal medium is an ImmunoCult^{™}-branded basal medium.

In one embodiment, NK cells may be cultured in any media that supports their maintenance, growth or expansion. Examples of such media include, but are not limited to ImmunoCult^{™} NK Cell Expansion Kit, ImmunoCult^{™} NK Cell base medium, StemSpan^{™} NK Cell Generation Kit, and StemDiff^{™} NK Cell Kit (all STEMCELL Technologies). Such media may be supplemented with cytokines such as IL-2, IL-7, IL-15, IL-21 or combinations thereof.

In one embodiment, T cells may be cultured in any media that supports their maintenance, growth, or expansion. Examples of such media include, but are not limited to ImmunoCult^{™}-XF T cell expansion medium, StemSpan^{™} T Cell Generation Kit, and StemDiff^{™} T Cell Kit (STEMCELL Technologies). Such media may be supplemented with cytokines such as IL-2, IL-7, IL-15, IL-21 or combinations thereof. Such media may be further supplemented with T cell activation reagents such as ImmunoCult^{™} Human T cell activation reagents (STEMCELL Technologies).

Methods of this disclosure may comprise co-culturing effector cells and target cells in a suitable co-culture medium in the presence of a binding composition. The nature and concentration of a binding composition may be as described herein.

With regard to E/T cell ratios, an appropriate or optimal E/T cell ratio may need to be empirically determined. In the context of the methods disclosed herein, an effector to target (E/T) cell ratio may range from 1:1 to 20:1. In one embodiment, effector cells are co-cultured at a ratio of about 1:1 with target cells. In one embodiment, effector cells are co-cultured at a ratio of about 3:1 with target cells. In one embodiment, effector cells are co-cultured at a ratio of about 6:1 with target cells. In one embodiment, effector cells are co-cultured at a ratio of about 5:1 with target cells. In one embodiment, effector cells are co-cultured at a ratio of about 1.75:1 with target cells. In one embodiment, effector cells are co-cultured at a ratio of about 9:1 with target cells. In one embodiment, effector cells are co-cultured at a ratio of about 15:1 with target cells. In one embodiment, effector cells are co-cultured at a ratio of about 20:1 with target cells. Where an effector cell may be present in a mixed population of cells (e.g., PBMCs), an effector to target (E/T) cell ratio may range from 1:1 to 50:1, 1:1 to 100:1, 1:1 to 200:1, 1:1 to 300:1, 1:1 to 400:1, or 1:1 to 500:1.

Methods of this disclosure may further comprise assessing or measuring an effector function of effector cells. Depending on the nature of the assessment, the skilled person will know the type(s) of assays that are suitable for obtaining a desired read-out. For example, assessing or measuring effector function of effector cells may be performed by flow cytometry, marker expression analysis, cytokine production, protein analysis, transcriptomic analysis, or metabolomic analysis.

Where an effector function of effector cells is killing of target cells, killing (or apoptosis), such as of target cells, may be assessed or measured by Annexin V staining based methods, such as by flow cytometry. In addition or in the alternative, killing (or apoptosis), such as of target cells, may be measured or assessed in other ways, such as caspase expression/activation, cytochrome c translocation, propidium iodide (PI) staining, neutral red staining, cell size quantification, Lactate dehydrogenase (LDH) release, inflammatory marker expression, cell morphology, DAPI/Hoechst staining, analysis of ATP levels, DNA fragmentation analysis, Calcium (Ca²⁺) gradients, mitochondrial function/integrity analysis, (3-(4,5-dimethylthiazol-2-yl)-2,5-diphenyltetrazolium bromide (MTT) assay, or oxygen consumption analysis.

Where an effector function of effector cells is degranulation, such as of effector cells, degranulation may be assessed or measured by membrane expression of the lysosomal protein CD107, such as by flow cytometry.

Where an effector function of effector cells activates either target cells or effector cells, activation may be measured by analysis of surface markers, cytokine release, release of cytotoxic proteins, transcriptomic analyses, protein analyses and/or metabolomic analyses.

Thus, methods of this disclosure may offer an improvement over current state of the art methods of eliciting effector function of effector cells upon target cells, such as cell potency or killing assays. Without being bound by theory, physically linking effector cells and target cell via a binding composition of this disclosure, thereby forcing such cells into close proximity of one another, may enhance an effector function of effector cells compared to conditions where effector cells and target cells are not linked via a binding composition of this disclosure.

Methods of this disclosure may be applied to cell types that do not express Fc receptors (e.g., T cells or PSC-derived cell types that do not express Fc receptors compared to their primary-derived counterparts). Methods of this disclosure may therefore be used to bring together a diverse range of cell types into close proximity of each other, without being limited by a requirement of a specific antigen/marker (e.g., Fc receptors) on the surface of an effector or a target cell to elicit an effector function of an effector cell.

Methods of this disclosure may offer an advantage over potency assays known in the art to characterize PSC-derived cell types. An effector and/or a target cell differentiated from a PSC may have a variable, low or non-existent expression of an antigen (eg., a first or a second antigen) compared to its primary source-derived counterpart (for eg., a primary NK cell and a PSC-derived NK cell may have varying expression of an Fc receptor such as CD16) and may therefore not be amenable to conventional assays reliant on such an antigen. Methods of this disclosure may be used to characterize PSC-derived cell types irrespective/independent of a variable expression of an antigen between PSC-derived cell types and their primary counterparts and be widely applied to characterize primary or PSC-derived effector or target cells.

Methods of this disclosure may offer more flexibility in terms of a binding composition used to link an effector and a target cell. Where a first polypeptide member and a second polypeptide member are indirectly linked via at least a third polypeptide member, the first polypeptide member and/or the second polypeptide member may be easily changed depending on the cell type (e.g., a target cell or an effector cell) to be assessed. Such a flexibility in exchanging the first polypeptide member and/or the second polypeptide member may not be possible where the first polypeptide member and the second polypeptide member are directly conjugated or comprised in a bispecific antibody and involve expensive or time-intensive processes. Thus, methods of this disclosure may be more feasible, adaptable and economical compared to other methods known in the art.

Physical linking of an effector cell and a target cell via a binding composition of this disclosure may enhance an effector function of the target cell by about 5%, 6%, 7%, 8%, 9%, 10%, 15%, 20%, 25%, 30%, 35%, 40%, 45%, 50%, 55%, 60%, 65%, 70%, 75%, 80%, 85%, 90%, or 95% compared to when an effector cell and a target cell are co-cultured but not physically linked via the binding composition. Linking an effector cell and a target cell via a binding composition may enhance an effector function of the effector cell by at least 5%, at least 10%, at least 20%, at least 30% or more compared to when an effector cell and a target cell are co-cultured but not linked via the binding composition.

Physical linkage of an effector cell and a target cell using a binding composition of this disclosure may increase an activation or killing of the target cell about 2-fold, 3-fold, 4-fold, 5-fold, 6-fold, 7-fold, 8-fold, 9-fold, 10-fold, or more compared to when an effector cell and a target cell are co-cultured but not physically linked using the binding composition.

In one embodiment, a binding composition enhances an effector function of an effector cell by at least 5-90%, or 10 - 90%, or 15-90%, or 20-90%, or 25-90%, or 30-90%, or 35-90%, or 40-90%, or 45-90%, or 50-90%, or 55-90%, or 60-90%, or 65-90%, or 70-90%, or 75-90%, or 80-90% compared to the effector function of an effector cell co-cultured with a target cell but not linked to the target cell via the binding composition.

In one embodiment, a binding composition enhances an effector function of an effector cell by at least 30% compared to the effector function of an effector cell co-cultured with a target cell but not linked to the target cell by the binding composition.

Accordingly, physically linking effector cells and target cells (using a binding composition) may enhance an effector function of effector cells upon target cells.

In another aspect, this disclosure may provide methods of treating, preventing or modulating a disease in an individual in need thereof, comprising administering a binding composition to the individual, and linking *in vivo* endogenous or exogenous (e.g autologous or allogeneic) effector cells to target cells (e.g. tumor cells) via the binding composition. A binding composition of such an aspect may be directly linked or indirectly linked as described in the aspects above. Administration of the binding composition may result in an enhanced effector function of the effector cells such as killing or activation of target cells. In some embodiments, a disease may be a cancer or a tumor. An effector cell and a target cell of such an aspect may be as described in the aspects above. In some embodiments, an effector cell may comprise PBMCs, CD8⁺ T cells, macrophages, NK cells or Chimeric antigen receptor (CAR) T-cells. In some embodiments, a target cell may comprise an ovarian carcinoma cell, a breast adenocarcinoma cell, a lung carcinoma cell, a melanoma cell, a B cell lymphoma and/or leukemia cell, a myeloid leukemia cell, a multiple myeloma cell or a T cell leukemia cell.

### Compositions

In another aspect of this disclosure are provided compositions for eliciting an effector function of an effector cell. Binding compositions will physically link an effector cell to a target cell, and by bringing effector cells and target cells into close proximity of one another the likelihood of eliciting an effector function may increase relative to a reliance on effector cells and target cells coming into contact with one another by passive diffusion in a co-culture.

Binding compositions of this disclosure may comprise a first recognition motif of a first polypeptide member that binds a first antigen of an effector cell; and a second recognition motif of a second polypeptide member that binds a second antigen of a target cell, wherein the first polypeptide member and the second polypeptide member are indirectly linked. The nature of the first recognition motif and the second recognition motif may be as described herein, but all that is important is that they respectively interact with antigens of effector cells and target cells with sufficient strength/affinity to maintain these cells in a close proximity to one another.

A first polypeptide member and a second polypeptide member are linked or linkable to one another, whether directly or indirectly.

A composition of this disclosure may further comprise: (i) a scaffold, a bead or a particle (as described above); and/or (ii) at least a third polypeptide member (as described above) to indirectly link the first polypeptide member and the second polypeptide member.

A first polypeptide member and/or a second polypeptide member may be an antibody, an antibody fragment, an antibody mimetic, an immunoglobulin, or a protein. In one embodiment, a binding composition may be a bispecific antibody engineered to bind both a first antigen of an effector cell and a second antigen of a target cell, wherein each variable region of a bispecific antibody binds respectively to antigens of an effector cell and a target cell.

In one embodiment, a first recognition motif is comprised in a first polypeptide member (e.g. a first antibody or a fragment thereof) and a second recognition motif is comprised in a second polypeptide member (e.g. a second antibody or a fragment thereof).

Where a first polypeptide member and a second polypeptide member are indirectly linked via at least one third polypeptide member, linkage of the first polypeptide member and the second polypeptide may be intermediated by at least one or two third polypeptide member. If a first polypeptide member and a second polypeptide member of a binding composition are indirectly linked via at least one third polypeptide member reliant on antigen:recognition motif interaction, then an arising binding composition may comprise polypeptide members indirectly linked or held in an immunological complex or array. In a specific, and non-limiting embodiment, a first polypeptide member and a second polypeptide member are indirectly linked via at least one third polypeptide member, wherein the first and second polypeptide members comprise a full or partial Fc fragment and the at least one third polypeptide member comprises at least two recognition motifs that bind the full or partial Fc fragment of the first and second polypeptide members.

If directly linked, a first polypeptide member that binds a first antigen of an effector cell and a second polypeptide member that binds a second antigen of a target cell may be conjugated to one another using conventional chemistry/biochemistry approaches or fused to one another using conventional molecular biology approaches.

In some embodiments, a first polypeptide member that binds a first antigen of an effector cell and a polypeptide member that binds a second antigen of a target cell may be indirectly linked via a scaffold, a bead or a particle, as described above.

In one embodiment, a first recognition motif of a first polypeptide member and a second recognition motif of a second polypeptide member comprised in a binding composition bind the same antigen. Thus, such a binding composition may be referred to as a monospecific binding composition. In one embodiment, a monospecific binding composition comprises first and second antibodies (or fragments) that recognize the same antigen on effector cells and target cells.

In one embodiment, a first recognition motif of a first polypeptide member and a second recognition motif of a second polypeptide member comprised in a binding composition bind/recognize different antigens. Thus, such a binding composition may be referred to as a bispecific binding composition. In one embodiment, a bispecific binding composition comprises a first antibody (or fragment) that binds/recognizes a first antigen on an effector cell and a second antibody (or fragment) that binds/recognizes a second antigen on a target cell.

First and second antigens of this disclosure are not particularly limited, and may be as described elsewhere herein.

Binding compositions of this disclosure may comprise one or more protein component each at a concentration within the range of 10 -1000 ng/ml. In one embodiment, each protein component (e.g. antibody or fragment thereof) in a binding composition is present at a concentration within the range of 10 - 600 ng/ml.

Binding compositions of this disclosure may offer an improvement over current state of the art compositions for eliciting effector function of effector cells upon target cells, such as cell potency or killing assays. Binding compositions of this disclosure may offer more flexibility in terms of the types of effector cells and target cells that may be brought into close proximity of one another. Binding compositions of this disclosure may be modified easily to accommodate different types of recognition motifs that bind to different types of antigens thus significantly broadening the repertoire of cell types that could be physically linked to elicit appropriate effector function of effector cells such as killing or activation of target cells. Binding compositions of this disclosure with multiple specificities (directed towards one or more antigen) may be easier and cheaper to produce than bifunctional antibodies or other antibody formats known in the art. Binding compositions of this disclosure may be used to characterize PSC-derived effector or target cells.

In another aspect of this disclosure are provided compositions for use in a method of treating, preventing or modulating a disease in an individual in need thereof, the method comprising administering a composition to the individual, and linking *in vivo* endogenous or exogenous (e.g autologous or allogeneic) effector cells to target cells (e.g. tumor cells) via the composition.

A composition of such an aspect may be a binding composition as described above. A binding composition may comprise a first recognition motif of a first polypeptide member that binds a first antigen of an effector cell; and a second recognition motif of a second polypeptide member that binds a second antigen of a target cell, wherein the first polypeptide member and the second polypeptide member are indirectly linked, as described above. A composition may further comprise (i) a scaffold, a bead or a particle; and/or (ii) at least a third polypeptide member, to indirectly link a first polypeptide member and a second polypeptide member, as described above. Administration of the binding composition may result in an enhanced effector function of effector cells such as killing or activation of target cells in the individual. In some embodiments, a disease may be a cancer or a tumor. An effector cell, a target cell, a first antigen or a second antigen of such an aspect may be as described in the aspects above. In some embodiments, an effector cell may comprise PBMCs, CD8⁺ T cells, macrophages, NK cells or Chimeric antigen receptor (CAR) T-cells. In some embodiments, a target cell may comprise an ovarian carcinoma cell, a breast adenocarcinoma cell, a lung carcinoma cell, a melanoma cell, a B cell lymphoma and/or leukemia cell, a myeloid leukemia cell, a multiple myeloma cell or a T cell leukemia cell. Accordingly, in some embodiments, the cancer or tumor to be treated, prevented or modulated may be selected from the group consisting of: an ovarian carcinoma; a breast adenocarcinoma; a lung carcinoma; a melanoma; a B cell lymphoma; a B cell leukemia; a myeloid leukemia; a multiple myeloma; and a T cell leukemia. Suitably, treatment, prevention or modulation comprise one or more effector functions (as described in more detail elsewhere in the specification) selected from the group consisting of: killing a target cell (e.g. by activating apoptotic pathways, such as through death receptors, recruitment of specialized adaptor proteins, or degranulation); activating effector cells (e.g. by inducing production of cytokines, expression of surface markers, metabolomic changes in effector cell, or changes in their transcriptomes); activating a target cell (e.g. inducing release of cytokines, chemokines or antibodies, activation of receptors, induction of signaling or transcription pathways and induction of mRNA or protein expression); and activating a target cell and cytotoxic degranulation (which may be assessed by membrane or surface expression of lysosomal-associated proteins) in the treatment, prevention or modulation.

The following non-limiting examples are illustrative of the present disclosure.

### Examples

### Example 1: Preparation and culture of cells

Effector NK cells were isolated from leukapheresis samples of peripheral blood mononuclear cells (PBMCs) by immunomagnetic negative selection using the EasySep^{™} Human NK Cell Isolation Kit (STEMCELL technologies). Isolated NKs were cultured in ImmunoCult^{™} NK Cell Base Media (STEMCELL Technologies), as directed by the manufacturer, with or without IL-2 or IL-15 cytokine supplements.

Target NALM-6 cells were purchased from DSMZ (DSMZ-German Collection of Microorganisms and Cell Cultures GmbH) and were maintained in media, as per vendor's instructions.

Effector Pan T cells and CD8⁺ T cells were isolated from leukapheresis samples using EasySep^{™} Human T Cell Isolation Kit (STEMCELL Technologies) and EasySep^{™} Human CD8⁺ T Cell Isolation Kit (STEMCELL Technologies), respectively. Isolated T cells or CD8⁺ T cells were treated with ImmunoCult^{™} Human CD3/CD28/CD2 T cell activator (STEMCELL Technologies) and cultured in ImmunoCult^{™}-XF T cell expansion medium (STEMCELL Technologies), as directed by the manufacturer, in the presence of cytokines such as IL-2, IL-7, IL-15 or IL-21 for 8 to 12 days.

### Example 2: Preparation of Binding Compositions

The following protocol may be used to prepare an embodiment of the binding composition for use in the methods of the present disclosure: A first polypeptide member (e.g., monoclonal antibody) recognizing a specific cell surface antigen/marker on effector cells (e.g. CD3, CD56, CD335/NKp46, or an Fc receptor) was mixed with a second polypeptide member (e.g., monoclonal antibody) recognizing a specific cell surface antigen/marker on target cells (e.g. CD19, Fc receptor, etc.). Binding compositions of the present disclosure spontaneously form when the above mentioned first and second polypeptide members (e.g., antibodies) are mixed with a third polypeptide member (e.g., antibody) that links the first polypeptide member and the second polypeptide member, such as by binding the Fc-regions thereof.

### Example 3: Fluorescent labelling of Target cells

Target cells (e.g. NALM-6) were cultured as per cell line specifications. Around 5×10⁶ target cells were harvested, washed twice with PBS and resuspended in prewarmed PBS at 2X the final concentration of cells (≤ 1×10⁷ cells/mL) required for labelling. For cell counts less than 5×10⁶ cells, 0.5mL of PBS was used. The recommended maximum final concentration of cells is 1×10⁷ cells/mL prior to the addition of dye. Amine-reactive cellular dyes were used for labelling, such as eBioscence^{™} Cell Proliferation Dye eFluor^{™} 670nm (Invitrogen^{™}) or CellTrace^{™} Violet Proliferation Dye (Invitrogen). A working solution of proliferation dye was prepared using prewarmed PBS at a dilution ranging from 1/500 to 1/2000, preferably 1/1000. The dye solution was added to a suspension of target cells at a 1:1 volume ratio such that the maximum final concentration of cells was ≤ 1×10⁷ cells/mL. The solution was mixed 2-3 times and incubated for 10 minutes, protected from light, to label the cells The excess dye label was quenched by adding 4 - 5 volumes of ice cold PBS or media, supplemented with 10% FBS. The target cell suspension was incubated on ice for 5 minutes, protected from light, to ensure removal of dye not incorporated by target cells. Labelled target cells were then centrifuged at 1200 rpm for 10 minutes and the pellet was resuspended in 10mL of ImmunoCult^{™}-XF medium and centrifuged again. Finally, the cells were resuspended in 1mL of co-culture medium as described below.

### Example 4: Co-culture of Effector and Target cells

For co-culture experiments to assess effector function of T or NK cells, target cells were labelled with an amine-based cellular dye prior to co-culture with NK or T cells to distinguish between effector cells and target cells, as described in Example 3. The target cells (e.g. NALM-6) were added to U-bottom 96W TC plates at 10,000 cells per well. The effector cells (NK or T cells) were added at a variety of cell concentrations to establish an effector function curve, typically ranging from 3000 to 100,000 per well. Binding compositions were added at a range of final volumes (0, 2, 4, 6, 8 and 10 µL/well) corresponding to a range of final concentration (50 ng/ml - 500 ng/ml of each polypeptide member) to establish an effective dose range. Co-cultures were incubated for 4 - 6 hours prior to measuring cell death. ImmunoCult^{™} NK cell base and ImmunoCult^{™}-XF T cell expansion media (STEMCELL Technologies) were used as the co-culture medium for NK and T cell killing assays, respectively. After co-culture, the cells were harvested and stained with a viability dye and an anti-human NK or a T cell marker-specific fluorescently conjugated antibody. Fluorescently labelled targets were evaluated for levels of Annexin V and viability dye staining on a flow cytometry analyzer to determine % target cell death. The frequency of stimulated NK or T cells was evaluated by assessing CD107 staining on cells that were stained with a marker for NK or T cells (and are negative for the fluorescent dye used to label the target cells).

### Example 5: Characterisation of Effector function by flow cytometry

### i) Annexin V staining

In order to determine the level of spectral overlap between different fluorophores and to compensate for this overlap, single staining controls were prepared and plated in the same plate as the co-culture of effector and target cells. Unlabelled targets were used as the unstained control. Unlabelled target cells mixed with effector cells, heat-killed unlabelled target cells, and labelled target cells were used as flow cytometry compensation controls for Phycoerythrin (PE), 7AAD and eFluor^{™} 670 dye, respectively. Fluorescence minus one (FMO) controls, FMO-PE and FMO-7AAD, of labelled target cells with effector cells were also included.

The effector cells (e.g. T/NK cells) and target cells were co-cultured for desired time points (usually 4-6 h) as described in Example 4. After co-culture, plates were then centrifuged and the supernatant was gently decanted with multi-channel pipettes. The cells were washed twice with cold PBS (180uL per well) and resuspended in 80uL of 1x Annexin V Binding Buffer (Tonbo Biosciences / Cytek #TNB-5000-L050 or prepared in-house) with 0.2uL PE-conjugated Annexin V reagent (Tonbo Biosciences/Cytek #50-6409-T100) and 1/250 7AAD (Tonbo Biosciences/Cytek # 13-6993-T200). Target cells without any effector cells added (spontaneous death) and effector cells without any target cells were both used as assay controls for flow cytometry-based characterisation.

### ii) Degranulation Staining

Degranulation of effector cells was detected by adding a PE-conjugated anti-human CD107 antibody (0.1-0.25 uL/well) at the start of effector/target cell co-culture. 0.1 uL of a protein transport inhibitor containing Monensin (BD GolgiStop^{™} Protein Transport Inhibitor, Catalog No. 554724) was added to each well at 1 -2 hours of co-culture. Cells were then co-cultured for an additional 4-5 hours, as described in Example 4, after which the plates were centrifuged and the supernatant was carefully decanted. Optionally, FcR blocking reagents (STEMCELL Technologies) were added to block Fc receptors prior to analysis of surface markers for NK cells (CD16 or CD56) or T cells (CD8 or CD3). A viability dye such as 7AAD or Draq7 was used if cells were analysed immediately, otherwise a fixable viability dye was added if the cells were going to be fixed and analysed at a later time. Staining controls including single stains and FMO controls as described above were included as needed. Effector cells cultured without target cells (spontaneous death) was used as a control. Phorbol 12-myristate 13-acetate (PMA) or ionomycin were used as positive controls. NK or T cell marker expression were gated and %CD107 staining was evaluated among the viable cells in the presence or absence of target cells or binding compositions.

### Example 6: Assessing effector function of NK cells

Effector NK cells and target NALM-6 cells were cultured in accordance with Example 1. Target cells were fluorescently labelled and co-cultured with Effector NK cells as described in Examples 3 and 4. Binding compositions comprising a first polypeptide member (e.g., antibody) against an NK cell marker/antigen (e.g. CD16, CD56 or NKp46) indirectly linked to a second polypeptide member (e.g. antibody) against a NALM-6 cell marker/antigen (e.g. CD19) were prepared in accordance with Example 2. Two different volumes of the binding composition (1µL & 4 µL) were tested at an E/T ratio of 3:1. Cells co-cultured without any binding composition were used as a control.

Target cell death was assessed by Annexin V based flow cytometry as described in Example 5. The percentage of Annexin V⁺ target cells was higher in the presence of the binding composition for both tested binding composition volumes compared to co-culture in the absence of a binding composition (Figure 1). These results indicate that all tested binding compositions were effective in inducing the killing function of NK cells in comparison to killing assays performed in the absence of a binding composition. While all binding compositions showed higher killing compared to a control, those binding composition comprising anti-CD16 and anti-CD19 antibodies showed the highest levels (40 -60%) of target cell death. Further, 1µL of the binding composition (comprising about 50 ng/ml of each polypeptide member) was found to be sufficient to induce effective target cell death.

### Example 7: Assessing effector function of Activated T cells

Day 10 expanded effector pan T cells prepared in accordance with Example 1 were co-cultured with fluorescently labelled target NALM-6 cells for 4 hours in the presence of a binding composition comprising a first polypeptide member (eg., anti-CD3 antibody) indirectly linked to a second polypeptide member (e.g. anti-CD19 antibody) (0.025 µg/ml), as described in Examples 2-4. Cell death of NALM-6 cells brought about by the effector function of T cells was assessed by flow cytometry expression of Annexin V⁺ cells, as described in Example 5. E/T ratios of 9:1, 6:1 and 3: 1 were tested.

As shown in Figure 2, addition of a binding composition comprising a first polypeptide member (e.g. an antibody) that binds a T cell antigen (e.g. CD3) indirectly linked to a second polypeptide member (e.g. an antibody) that binds a target cell antigen (e.g. CD19) (a CD3-CD19 binding composition) greatly induced target cell death compared to the baseline conditions, either in the absence of a binding composition (No binding comp.) or in the presence of an irrelevant binding composition (anti-dextran). Increasing levels of effector function (e.g. target cell death) were observed for E/T ratios up to 3:1, above this E/T ratio only a relatively small increase in target cell death was observed. This indicated that the effector Pan T cells isolated in accordance with Example 1 were highly active and relatively pure and could achieve about 60% killing of target cells at a relatively lower E/T ratio of 3:1 in the presence of the binding composition. T cells & NALM-6 cells co-cultured in the presence of an irrelevant binding composition (e.g. comprising indirectly linked first and second polypeptide members that bind dextran) displayed similar target cell death as the condition without any binding composition present, indicating that the observed high levels (>25%) of target cell death is achieved specifically by the binding composition that links effector cells to target cells.

### Example 8: Assessing effector function of Activated T cells in the presence of cytokines

Day 12 expanded effector T cells prepared in accordance with Example 1 were co-cultured with fluorescently labelled target NALM-6 cells for 4-5 hours in the presence of a binding composition comprising a first polypeptide member (e.g. an antibody) that binds a T cell antigen (e.g. CD3) indirectly linked to a second polypeptide member (e.g. an antibody) that binds a target cell antigen (e.g. CD19) (a CD3-CD19 binding composition) as per the methods described in Examples 2-4. Cells were co-cultured in the presence of different cytokines or cytokine combinations as follows: (i) IL-2, (ii) IL-2 + IL-21, (iii) IL-15, and (iv) IL-15 + IL-21. The foregoing cultures were done either in the presence (With CD3-CD19) or the absence (No CD3-CD19) of a binding composition as described in this paragraph.

High levels of target cell death (>50% or 60%) was observed in the presence of the binding composition having specificity for CD3 and CD19 (as described above) compared to conditions where target cells and effector cells were co-cultured in the absence of such binding composition (Figure 3A). The percentage of target cell death was comparable (about 60% to about 80%) for all individual or combinations of cytokines tested across all tested E/T ratios (Figure 3A). Expressed in a different graph, it is better apparent that conditions with cytokine combinations (IL-2/IL-15 + IL-21) displayed slightly higher target cell death by about 1-8% compared to individual cytokines (Figure 3B). A qualitative microscopic analysis of T cell and NALM-6 co-cultures in the presence of IL-2 and a binding composition as used to generate the data shown in Figures 3A and 3B, at an E/T ratio of 15:1, showed the formation of very tight clusters of cells compared to those lacking any binding composition (Figure 3C). This result demonstrated that the binding composition links the effector and target cell and brings them into close physical proximity to one another (Figure 3C, right panel) compared to when the effector and target cells are not linked via a binding composition (Figure 3C, left panel).

### Example 9: Assessing effector function of Cytotoxic CD8⁺ T cells in the presence of cytokines

CD8⁺ T cells were isolated and expanded over 8 days as described in Example 1, in the presence of different cytokines or cytokine combinations: IL-2, IL-7+15, or IL-21. Such cytokine (combinations) were either included in ImmunoCult^{™}-XF medium during expansion (Rest) or were supplemented 2 days prior to co-culture (Additional). CD8⁺ effector T cells were co-cultured with NALM-6 target cells in the presence of different volumes (1 µL/well to 10 µL/well) of a binding composition comprising a first polypeptide member (e.g. an antibody) that binds a T cell antigen (e.g. CD3) indirectly linked to a second polypeptide member (e.g. an antibody) that binds a target cell antigen (e.g. CD19) (a CD3-CD19 binding composition) at 3:1 or 1:1 E/T ratios. Target cell death and degranulation brought about by effector CD8⁺ T cells were measured as described in Example 5.

An E/T ratio of 3:1 resulted in about 10% or greater than about 10% Annexin V⁺ cells when IL-2 and IL-15 were supplemented 2 days prior to co-culture compared to when IL-2, IL-15 or IL-21 were added during expansion of effector T cells (Figure 4A). Further, 1-2 µL/well of the binding composition (corresponding to approximately 50-150 ng/ml of each polypeptide member) was sufficient to achieve greater than 50% of levels of target cell death. Further, addition of IL-15 2 days prior to co-culture under these conditions led to the highest percentage of Annexin V⁺ cells (60% or greater than about 60%).

Similarly, an E/T ratio of 1:1 resulted in about 2%-10% or greater than about 2%-10% Annexin V⁺ cells when IL-2 and IL-15 were supplemented 2 days prior to co-culture compared to when IL-2, IL-15 or IL-21 were added during expansion of effector T cells (Figure 4B). Although addition of IL-21 2 days prior to co-culture led to an increase in target cell death compared to cells without fresh addition of IL-21, the increase was not as proportionally high as observed for IL-2 and IL-15. Overall, higher levels of Annexin V⁺ cells were observed with an E/T ratio of 3: 1 compared to an E/T ratio of 1:1 (Figures 4A and 4B).

CD8⁺ T cell activation and cytotoxic degranulation was further assessed by flow cytometry-based membrane expression of the lysosomal protein CD107, as described in Example 5. As described above, 1-2 µL/well of the binding composition (corresponding to about 50 to 150 ng/ml of each polypeptide member in the binding composition) was sufficient to achieve cytotoxic degranulation of effector cells. Fresh addition of IL-15 2 days prior to co-culture resulted in 20% or higher than 20% cells expressing CD107⁺ cells (Figure 4C). Fresh addition of IL-21 2 days prior to co-culture also showed a marked increase in % of CD107⁺ cells compared with baseline conditions without fresh addition of IL-21 prior to co-culture, especially at 5 µL/well (corresponding to about 250 ng/ml of each polypeptide member) of the binding composition. These results demonstrated that addition of fresh cytokines prior to the co-culture of effector and target cells could further enhance the effector function of the effector cells in the methods of this disclosure.

### Example 10: Assessing effector function of PSC-derived Cytotoxic CD8⁺ T cells

H9 PSC lines were differentiated into CD8 single-positive (SP) T cells using the STEMdiff^{™} T Cell Kit (STEMCELL Technologies) as per Manufacturer's instructions. Four effector cell test samples were prepared corresponding to 4 different differentiations of H9 PSC lines. Target NALM-6 cells were fluorescently labelled and co-cultured with effector SP CD8⁺ T cells as described in Examples 3 and 4. Binding compositions comprising a first polypeptide member (e.g. an antibody) that binds a CD8⁺ T cell antigen (e.g. CD3) indirectly linked to a second polypeptide member (e.g. an antibody) that binds a target cell antigen (e.g. CD19) (CD3-CD19), were prepared in accordance with Example 2. Cells co-cultured without any binding composition were used as a control. Target cell death was assessed by Annexin V based flow cytometry as described in Example 5.

Without being bound by theory, it appeared that PSC-derived CD8⁺ T cells had a marked level of baseline potency and exhibited appreciable effector function (e.g. target cell death) even in the absence of the binding composition for all samples tested (Figure 5). Regardless, effector function of PSC-derived CD8⁺ T cells was enhanced when such cells were coupled with target cells using a binding composition as described in this Example. In some cases, effector function could be enhanced >10% in comparison to a co-culture of PSC-derived CD8⁺ effector T cells and target cells in the absence of a binding composition. Across all samples, effector function was increased by about 4-12% among PSC-derived CD8⁺ effector T cells co-cultured with and linked to target cells via a binding composition in comparison to PSC-derived CD8⁺ effector T cells co-cultured with but not linked to target cells via a binding composition. These results indicate that the binding compositions can elicit an enhanced effector function of PSC-derived cytotoxic CD8⁺ T cells.

While the present disclosure has been described with reference to what are presently considered to be the preferred examples, it is to be understood that the disclosure is not limited to the disclosed examples. To the contrary, the disclosure is intended to cover various modifications and equivalent arrangements included within the spirit and scope of the appended claims.

All publications, patents and patent applications are herein incorporated by reference in their entirety to the same extent as if each individual publication, patent or patent application was specifically and individually indicated to be incorporated by reference in its entirety.

## Claims

1. A method of eliciting an effector function of an effector cell, the method comprising:
co-culturing the effector cell displaying a first antigen and a target cell displaying a second antigen in the presence of a binding composition comprising a first recognition motif of a first polypeptide member that binds the first antigen and a second recognition motif of a second polypeptide member that binds the second antigen;
linking the effector cell and the target cell via the binding composition; and
eliciting the effector function of the effector cell.

2. The method according to claim 1, wherein the effector cell does not comprise an Fc receptor.

3. The method according to claim 1 or 2, wherein the effector cell is an immune cell preferably a T cell or any T-cell subtype, an NK cell or any NK cell subtype, or an NKT cell.

4. The method according to any one of claims 1 to 3, wherein the target cell is a cell or a mass of cells, and the mass of cells is a tumor, an organoid or a spheroid.

5. The method according to any one of claims 1 to 4, wherein the effector function of the effector cell comprises killing the target cell or activation of the effector or target cell.

6. The method according to any one of claims 1 to 5, wherein co-culturing the target cell and the effector cell is for a time period sufficient to a) link the effector cell and the target cell, and/or b) elicit the effector function of the effector cell, wherein the time period ranges between 0.5 to 24 hours.

7. The method according to any one of claims 1 to 6, wherein co-culturing the effector cell and the target cell further comprises IL-2, IL-15, IL-21 or a combination thereof.

8. The method according to any one of claims 1 to 7, wherein linking the effector cell and the target cell via the binding composition enhances the effector function of the effector cell by at least 5%, at least 10%, at least 20%, at least 30% or more compared to when an effector cell and a target cell are co-cultured but not linked via the binding composition.

9. The method according to any one of claims 1 to 8, wherein the target cell and/or the effector cell are adherent cells or suspension cells.

10. The method according to any one of claims 1 to 9, wherein the first polypeptide member and the second polypeptide member are directly linked.

11. A composition, comprising:
a first recognition motif of a first polypeptide member that binds a first antigen of an effector cell; and
a second recognition motif of a second polypeptide member that binds a second antigen of a target cell,
wherein the first polypeptide member and the second polypeptide member are indirectly linked.

12. The method of any one of claims 1 to 9, or the composition of claim 11, further comprising:
(i) a scaffold, a bead or a particle; and/or
(ii) at least a third polypeptide member,
to indirectly link the first polypeptide member and the second polypeptide member.

13. The method of any one of claims 1 to 10, or the composition according to claim 11 or 12, wherein the first polypeptide member and/or the second polypeptide member is an antibody, an antibody fragment, an immunoglobulin or a protein.

14. The method of any one of claims 1 to 10, or the composition according to any one of claims 11 to 13, wherein the first antigen is comprised in CD3, CD16, CD56, CD335/NKp46 or a receptor on the surface of the effector cell.

15. The method of any one of claims 1 to 10, or the composition according to any one of claims 11 to 14, wherein the second antigen is comprised in CD19, a tumor antigen or a receptor on the surface of the target cell.
